# EUROPÄISCHE PATENTSCHRIFT

(11) **EP 1 531 182 B1**
(45) Veröffentlichungstag und Bekanntmachung des Hinweises auf die Patenterteilung: **21.10.2009**
(21) Anmeldenummer: 04026275.0
(22) Anmeldetag: 05.11.2004
(51) Int. Cl.: C12P 7/64, C12P 33/00

(54) **Verfahren zur Gewinnung von Sterolen und polaren Lipiden aus Lecithin durch Ultrafiltration und Vorbehandlung mit Amylasen und Glucosidasen**
Process for obtaining sterols and polar lipids from lecithin by ulrafiltration and pretreatment by amylases and glucosidases
Procédé d'obtention de stérols et de lipides polaires à partir de lécithine par ultrafiltration et prétraitement par amylases et glucosidases

(30) Priorität: 14.11.2003 DE 10353150
(43) Veröffentlichungstag der Anmeldung: 18.05.2005
(73) Patentinhaber: Cognis IP Management GmbH, 40589 Düsseldorf (DE)
(72) Erfinder: Both, Sabine, Dr., 41470 Neuss (DE); Alexandre, Teresa, 47802 Krefeld (DE); Gutsche, Bernhard, 40724 Hilden (DE); Kray, Jürgen, 49764 Langenfeld (DE); Beverungen, Carsten, 40591 Düsseldorf (DE); Eickenberg, Rainer, 40233 Düsseldorf (DE)

(56) Entgegenhaltungen:
- EP-A- 0 049 914
- US-A- 4 062 882
- US-A- 4 399 224
- US-A- 6 140 519

## Beschreibung

### Gebiet der Erfindung

Die Erfindung befindet sich auf dem Gebiet der oleochemischen Rohstoffe und betrifft die gekoppelte Gewinnung verschiedener Wertstoffe aus den Rückständen der Raffination pflanzlicher Öle.

### Stand der Technik

Zu den Begleitstoffen pflanzlicher Öle, wie beispielsweise Soja-, Raps-, Sonnenblumen-oder Leinöl, gehören Phosphatide, Proteine, Kohlenhydrate, Schleimstoffe sowie kolloidale Verbindungen, die die Haltbarkeit des Öls stark herabsetzen und die hydrolytische und oxidative Fettspaltung fördern. Für die Raffination sind sie hinderlich, da sie den Raffinationsverlust stark erhöhen. Auf andere Operationen üben sie ebenfalls negativen Einfluss aus. So behindern sie beispielsweise die Kristallisation bei der Fraktionierung und verstopfen die Poren des Katalysators bei der Härtung. Im fertigen Öl würden sie sich mit der Zeit als Bodenschlamm absetzen und es so verdorben erscheinen lassen. Aus all diesen Gründen werden bestimmte Öle, die einen nennenswerten Gehalt dieser Stoffe haben, entschleimt. Unter Entschleimen versteht man in diesem Zusammenhang das Entfernen der gesamten Gruppe dieser Stoffe, unabhängig davon, ob es sich tatsächlich um Schleimstoffe handelt oder nicht. Die Entschleimung kann auf unterschiedlichem Wege erfolgen. Ein Teil der Phosphatide lässt sich beispielsweise abtrennen, indem man sie hydratisiert. Dadurch verlieren sie ihren lipophilen Charakter, fallen aus dem Öl aus und können abgetrennt werden. Nicht hydratisierbare Phospholipide werden mit Säuren zerstört und anschließend mittels Separation aus dem Öl entfernt. Die Säure muss dabei gerade so stark sein, dass sie die Phospholipide spaltet, das Öl aber nicht angreift. Für diesen Zweck werden überwiegend Phosphor- oder Zitronensäure verwendet. Einige Öle wie z. B. Leinöl können auch hitze-entschleimt werden. Bei diesem Prozess, den man auch als "Brechen des Öls" bezeichnet, wird das Ausgangsmaterial auf 240 bis 280 °C erhitzt, wobei die Schleimstoffe ausfallen und können abgetrennt werden können. Nichtsdestotrotz enthalten die bei der Entschleimung anfallenden Lecithinfraktionen wertvolle Rohstoffe, die für weitere Anwendungen im Bereich Kosmetik, Pharma und Nahrungsmittel Einsatz finden können. So liegt der Gehalt an den verschiedenen Phospholipiden in technischen Lecithinfraktionen in der Regel bei etwa 50 bis 55 Gew.-%, an Sterolen und Sterolderivaten bei 1 bis 10 Gew.-%, an neutralen Lipiden bei 30 - 40 Gew.-% und an Ceramiden und Cerebrosiden bei 0,1 bis 0,5 Gew.-%.

Problematisch ist jedoch, dass die Mengen an Wertprodukten, insbesondere an freien Sterolen und Ceramiden sehr unterschiedlich und im allgemeinen gering sind und daher aufwendige Reinigungs- und Konzentrierungsverfahren erforderlich werden, die ein ökonomisches Arbeiten in der Regel nicht erlauben.
- Aus dem Stand der Technik sind in diesem Zusammenhang verschiedene Lösemittelverfahren zur Anreicherung von Phospholipiden aus hydratisierbaren Lecithinen bekannt, bei denen Aceton als Extraktions- bzw. Fällungsmittel verwendet wird. Diesen Vorgang nennt der Fachmann: Entölung des Lecithins. Dabei wird das Rohlecithin mittels Aceton extrahiert, um die ca. 30-40 % neutralen Lipide zu entfernen. Man erhält sowohl pulverförmige als auch granulierte Produkte mit einem Rest-Triglyceridgehalt von ca. 1-2 %. Nachteilig ist, dass der Einsatz von Aceton aufwendig ist und die Produkte nur eine begrenzte Reinheit aufweisen. Da Aceton ein sehr untypisches Lösemittel in der ölverarbeitenden Industrie ist, stellte sich bereits Ende der 80er Jahre den Produzenten von Lecithinprodukten die Frage, ob die Zulassung dieses Lösungsmittels auch für die Zukunft gesichert ist.
- Auch Verfahren zur Aufarbeitung von Rückständen der Entschleimung durch Ultrafiltration sind grundsätzlich bekannt. In der US 4,093,540 (Lever Brothers) und in der US0116747 (The Texas A&M University System) wird vorgeschlagen, zur Anreicherung von Wertstoffen pflanzliches Öl einer Ultrafiltration durch eine Membran zu unterwerfen. Allerdings wird hier das gesamte ÖL und nicht das Lecithin durch eine Membran geschickt. Gegenstand der EP 0049914 A1 (Unilever) ist die Aufreinigung von Lecithins mittels Ultrafiltration. Die Aufreinigung von Lecithinen mittels Filtration durch eine semipermeable Membran ist auch Gegenstand der Japanischen Schrift JP 62-039594 (Rinoru). Auch aus der US 6,140,519 (Archer Daniels) ist ein Ultrafiltrationsverfahren bekannt, mit dessen Hilfe aufgereinigte Phosphatide hergestellt werden können. Keines der Verfahren bietet jedoch eine zusammenhängende Lehre gemäß der die unterschiedlichen Wertstoffe in einer technisch leicht durchführbaren Weise unter ökonomischen Bedingungen mit hinreichender Ausbeute und Reinheit herzustellen wären.
- Ebenfalls bekannt sind Verfahren zur Fraktionierung von entölten Lecithinen. Dabei unterscheidet man zwischen drei Verfahrensvarianten.:
   (a) Im *Lösemittelverfahren* werden die phosphatidhaltigen Gemische in Hexan oder Aceton gelöst und mit 50 Vol.-% Ethanol versetzt. Dabei bilden sich zwei Phasen aus: In der schwereren Phase befindet sich hauptsächlich Phosphatidylinositol/Phosphatidylethanolamin in der leichteren Phase im wesentlichen Phosphatidylcholin/Phosphatidylethanolamin, **[vgl.** Bailey's Industrial Oil & Fat Products, Volume 1, Edible Oil & Fat Products: General applications**].** Nachteilig bei diesem Verfahren ist die geringe Anreicherung einer Phospholipidkomponenente.
   (b) Mittels *chromatographischer Trennverfahren,* wie z.B. präparativer HPLC, lassen sich sehr zwar reine Fraktionen gewinnen, allerdings sind die Herstellkosten so hoch, dass eine wirtschaftliche Durchführung nicht möglich ist.
   (c) In den Druckschriften DD 27546 (Humboldt-Universität, Berlin), WO 83/003620 (Unilever) und EP 0049914 A1 (Unilever) werden Verfahren beschrieben, bei denen die Phospholipide mittels Membranen, d.h. *Ultrafiltration,* und Zugabe von polaren Lösungsmitteln in Fraktionen getrennt werden. Der Wirkstoffgehalt in den Fraktionen ist jedoch vergleichsweise gering, zudem wird die Gewinnung von Sterolen oder Sterolderivaten nicht in Betracht gezogen.
- *Extraktionsverfahren* zur Gewinnung von Sterolen gehören in diesem Zusammenhang ebenfalls zum Stand der Technik. Gegenstand der Druckschrift US 2,415,313 (Refining Unincorporated Housten) ist ein Verfahren zur Anreicherung von Sterolen und Sterolglykosiden, bei dem entsprechende pflanzliche Öle einer Lösemittelextraktion unterworfen werden. Über Reinheit und Ausbeuten werden indes keine Angaben gemacht. Gemäß der Lehre der US 2,445,931 (US Secretary of Agriculture) wird vorgeschlagen, den pflanzlichen Ölen Alkohole zuzusetzen und auf diesem Wege einen Feststoff zu fällen, von dem man allerdings nur vermutet, dass er Sterole und Lecithine enthält. Schließlich wird in Rev.Franc.Corps Gras 5, p307-315, (1958**)** über eine Extraktion nicht-hydratisierbarer Phosphatide mit Aceton berichtet. Die Mengen an so gewonnenen Sterolen lag jedoch mit 0,3 % denkbar schlecht. Auch diese Verfahren bieten somit keine Lehre an, wie man gleichzeitig verschiedene Wertstoffe aus Lecithinen bzw. lecithinhaltigen Ölschleimen in wirtschaftlicher Weise gewinnen kann.
- Stand der Technik ist ebenfalls die Aufarbeitung von Sterolen aus triglyceridhaltigen Gemischen. In der ersten Verfahrensalternative wird das komplette Gemisch mittels Alkalizugabe verseift und mit Lösungsmittel wie z. B. EDCL und/oder Heptan extrahiert. Nach einer Aufkonzentrierung werden die Sterole mittels Kühlungskristallisation gewonnen. In der zweiten Verfahrensaltemative wird das Gemisch mit Methanol in einer Hochdruckstufe umgeestert. Anschließend werden die erhaltenen Methylester abdestilliert, bis ca. 30 % freie Sterole im Rückstand enthalten sind. Diese werden dann mittels Kühlungskristallisation aus dem Methylestergemisch gewonnen.

US-A 4399224 beschreibt ein Verfahren zur Verflüssigung von pflanzlichen Lecithinen durch enzymatische Behandlung.

Die Aufgabe der vorliegenden Erfindung hat folglich darin bestanden, ausgehend von technischen Lecithingemischen bzw. lecithinhaltigen Rückständen aus der Entschleimung von pflanzlichen Ölen mit möglichst geringem technischen Aufwand und unter wirtschaftlich akzeptablen Bedingungen gleichzeitig Sterole und polare Lipide in hoher Reinheit zu gewinnen.

### Beschreibung der Erfindung

Gegenstand der Erfindung ist ein Verfahren zur gleichzeitigen Herstellung von Sterolen und polaren Lipiden, bei dem man Zubereitungen pflanzlicher Lecithine einer Ultrafiltration unterwirft und einerseits die Sterole im Permeat und andererseits die polaren Lipide im Retentat anreichert und anschließend in an sich bekannter Weise aufarbeitet, dadurch gekennzeichnet, dass man wässrige Lecithinzubereitungen zuvor mit Enzymen behandelt, die eine Amylase- und Glucosidase - Aktivität aufweisen.

Dabei erweist sich die Ultrafiltration als ein geeignetes Verfahren, um auch Wertstoffe aus solchen Lecithinfraktionen in hohen Ausbeuten und Reinheiten zur Verfügung zu stellen, die einen an sich geringen Gehalt an Sterolen, und polaren Lipiden, speziell Ceramiden und Phsopholipiden aufweisen.

### Ausgangsstoffe

Als Ausgangsstoffe für die Koppelproduktion kommen die Entschleimungsprodukte bzw. Raffinationsrückstände von pflanzlichen Ölen, wie beispielsweise Sojaöl, Rapsöl, Sonnenblumenöl, Leinöl, Linolaöl, Distelöl, Olivenöl und deren Gemischen in Frage. In gleicher Weise können natürlich auch Lecithinfraktionen aufgearbeitet werden, die aus anderen Quellen stammen.

### Unpolare Lösemittel

Polare Lipide und unpolare Lipide unterscheiden sich nur unwesentlich in ihren Molekülmassen, was die Trennung erschwert. In einer bevorzugten Ausführungsform der vorliegenden Erfindung werden den Lecithinlösungen daher vor der Ultrafiltration unpolare organische Lösemittel, vorzugsweise n-Hexan zugesetzt. Polare Lipide bilden im Gegensatz zu unpolaren Lipiden in Lösemitteln wie Hexan oder Aceton Micellen aus, was die Trennschärfe des Verfahrens wesentlich verbessert. Die Konzentration der Lecithine in den organischen Lösemitteln kann etwa 1 bis 30 Gew.-% betragen.

### Enzyme

Es werden wässrigen Lecithinzubereitungen Enzyme vom Typ der Glucosidasen und Amylasen bzw. mit Glucosidasen- und Amylasen-Aktivitäten zugesetzt, so dass alle im Gemisch enthaltenen Acetale zwischen Sterole und Glucose-Einheiten gespalten werden. Auf diesen Wege wird sichergestellt, dass die Sterolglucoside in freie Sterole und Zuckereinheiten überführt werden und die Ausbeute an Sterolen signifikant gesteigert wird. Als Enzyme können z.B. in Frage kommen: Depol® 40 L (Glucosidase von Biocatalysts), Depol® 220 L (Amylase, Biocatalysts), Cellubrix® (Glucosidase von Novozymes) und Novozym® 188 (Glucosidase von Novozymes), Beta-Glucosidase (Sigma) und alpha-Amylase (Sigma). Dabei kann die Einsatzkonzentration 0,01 bis 2, vorzugsweise 0,05 bis 0,5 Gew.-% Enzym bezogen auf Lecithin betragen. Die Enzyme werden in wässrige Lecithine mit 10 bis 60 Gew.-% Wasser gegeben. Die Reaktion findet üblicherweise bei Normaldruck zwischen 20 und 60 °C statt. Die Reaktionsdauer liegt typisch bei 10 bis 84 h. Nach Beendigung der Reaktion wird das Wasser vom Reaktionsgemisch z.B. durch Schwerkraft entfernt. Dadurch werden die von den Glucosiden abgespaltenen Glucoseeinheiten vom Lecithin getrennt. Der freie Sterolgehalt im verbleibenden Lecithin wird durch diese Behandlung um den Faktor 2 bis 6 angereichert. Das so behandelte und getrocknete Lecithin wird anschließend in einer Ultrafiltration weiter aufgetrennt.

### Durchführung des Verfahrens

Man behandelt zunächst wässrige Suspensionen pflanzlicher Lecithine mit Enzymen und entfernt die wässrige Phase nach erfolgter enzymatischer Reaktion. Im Anschluss daran werden die so behandelten Lecithine getrocknet und anschließend in einem unpolaren Lösemittel, vorzugsweise Hexan gelöst und einer Ultrafiltration mit nachgeschalteter Diafiltration unterworfen. Hierbei reichert man einerseits die freien Sterole und Sterolester im Permeat und andererseits polare Lipide wie z. B. Ceramide und Phospholipide im Retentat an. Anschließend werden beide Produktströme von dem Lösemittel befreit. Die verbleibenden Lipide im Permeat werden nach dem einschlägigen Stand der Technik verseift und die Sterole extrahiert sowie kristallisiert. Ebenso ist eine Umesterung der im Permeat verbleibenden Lipide mittels Methanol möglich. Durch gezieltes Destillieren kann zudem der Gehalt an Sterolen im Rückstand weiter erhöht werden; daran kann sich eine Kristallisation der Sterole anschließen. Die polaren Lipide können als Phospholipidfraktion oder mittels Fraktionierung per Ultrafiltration in angereichtere Phospholipide aufgearbeitet werden. Anschließend werden die im Retentat enthaltenen Ceramide mittels Lösemittelextraktion oder durch Zugabe von polaren Lösemittel und nachgeschalteter Ultrafiltration als angereicherte Fraktionen erhalten. Dabei werden die Micellen durch Zugabe von wenig polarem Lösemittel gebrochen. Dabei erweisen sich die Micellen der Ceramide weniger beständig als die der Phospholipide. Die erhaltenen Fraktionen werden anschließend von den Lösemitteln befreit und getrocknet. Falls gewünscht können die Phospholipide anschließend noch in die Fraktionen Phosphatidylinositol, Phosphatidylethanolamin und Phosphatidylcholin aufgetrennt werden.

### Ultra- bzw. Diafiltration

Unter dem Begriff Ultrafiltration versteht der Fachmann im allgemeinen Trennverfahren mittels poröse Membranen zur Abtrennung makromolekulare Stoffe mit Molekulargewichten im Bereich von 1×10³ bis 10⁶ Dalton bzw. einem Durchmesser im Bereich von 0,1 bis 0,001 µm. Im Sinne des erfindungsgemäßen Verfahrens soll der Begriff Ultrafiltration als Membranmikrofiltration verstanden werden, bei dem die unterschiedlichen Wertstoffe getrennt von einander im Permeat und Retentat angereichert werden.

Für die Wirtschaftlichkeit eines jenen Membranprozesses sind zwei Eigenschaften von zentraler Bedeutung: die Selektivität der Membran, d.h. ihre Fähigkeit, zwischen den Komponenten einer Mischung zu unterscheiden, und die Leistungsfähigkeit der Membran, d.h. der zu erzielende Permeatfluß unter bestimmten Betriebsbedingungen. Die Betriebsbedingungen für die Ultrafiltration orientieren sich an den Apparateabmessungen und insbesondere dem Membranmaterial. Typischerweise wird die Filtration bei Temperaturen im Bereich von 20 bis 50, vorzugsweise 30 bis 40 °C und Drücken im Bereich von 1 bis 10, vorzugsweise 3 bis 8 bar durchführt.

Wichtig bei dem Auswahl des Membranmaterials sind u.a. Porengrößenverteilung, Porosität, pH-Verträglichkeit, chemische und mechanische Stabilität, die der Fachmann durch routinemäßige Optimierung ermitteln kann ohne hierzu erfinderisch tätig werden zu müssen. Einige häufig eingesetzte Werkstoffe für UF-Membranen sind Polysulfon (PS), Polyethersulfon (PES), Celluloseacetat (CA), Polyamid (PA) und Polyvinylidenfluorid (PVDF). Hervorzuheben sind PVDF, das sich insbesondere durch gute Lösungsmittelverträglichkeit auszeichnet, sowie Polyethersulfon (PES), weil es wesentlich engere Porengrößenverteilungen als Polysulfon (PS) zulässt.

Die Ultrafiltration wird in Bauteilen durchgeführt, die als "Module" bezeichnet werden und dabei unterschiedliche Größen und Formen haben können. Bei der Modulauswahl muss ein Kompromiss gefunden werden, zwischen hoher Packungsdichte bei geringen Modulkosten einerseits und guter Modulspülbarkeit bzw. geringer Verblockungsneigung andererseits. Das Kissenmodul (Flachmembranen) hat die folgenden Vorteile: einfache, kostengünstige Fertigung, relativ hohe Packungsdichte und guter Stoffaustausch.

Diafiltration ist eine Variante der Ultrafiltration, die oft verwendet wird, um eine vollständigere Abtrennung zu erreichen. Bei der Diafiltration handelt es sich um die Verdünnung des Konzentrats mittels Zugabe von Lösungsmittel und anschließende Ultrafiltration bis die gewünschte Abtrennung des permeierden Stoffes erreicht ist. Das Lösemittel kann kontinuierlich zugeführt werden, um den Volumenverlust durch die Ultrafiltration zu kompensieren oder diskontinuierlich bei Verdünnung der Konzentration erreicht nach einer batchweisen Ultrafiltration.

Im Zuge der Ultrafiltration bzw. der Kombination aus Ultra- und Diafiltration, treten alle neutralen Lipide einschließlich der Sterinester und der freien Sterole, die zuvor freigesetzt wurden bzw. schon im Ausgangsstoff enthalten waren, in das Permeat über, während die polaren Lipide im Retentat verbleiben. Die sterolreichen Permeate werden gesammelt, vom Hexan befreit, und mit zwei Verfahrensaltemativen nach dem Stand der Technik aufgearbeitet. In der ersten Verfahrensalternative wird das komplette Gemisch mittels Alkalizugabe verseift und mit Lösungsmittel wie z. B. EDCL und/oder Heptan extrahiert. Nach einer Aufkonzentrierung werden die Sterole mittels Kühlungskristallisation in Reinheiten größer 90 % gewonnen.

In der zweiten Verfahrensaltemative wird das Gemisch mit Methanol in einer Hochdruckstufe umgeestert. Anschließend werden die erhaltenen Methylester abdestilliert, bis ca. 30 % freie Sterole im Rückstand enthalten sind. Diese werden dann mittels Kühlungskristallisation in Reinheiten größer 90 % aus dem Methylestergemisch gewonnen.

Anschließend werden die im Retentat enthaltenen polaren Lipide mittels Lösemittelextraktion oder durch Zugabe von polaren Lösemittel und nachgeschalteter Ultrafiltration als angereicherte Fraktionen erhalten. Dabei werden die Micellen durch Zugabe von - wenig - polarem Lösemittel gebrochen, wobei die Menge des Lösemittels bezogen auf das Retentat 0,1 bis 50, vorzugsweise 1 bis 15 und insbesondere 2 bis 10 Gew.-% betragen kann. Die Zugabe des polaren Lösungsmittel, z. B. eines aliphatischen C₁-C₆-Alkohols, vorzugsweise Ethanol oder Isopropylalkohol, erfolgt vorzugsweise stufenweise, damit die Phospholipide und die Ceramide nacheinander freigesetzt werden. In Stufe 1 werden zwischen 0,1 bis 3 Gew.-% polares Lösemittel zugegeben. Dabei erweisen sich die Micellen der Ceramide weniger beständig als die der Phospholipide. Diese werden bei der durchgeführten Ultrafiltration als erstes im Permeat wiedergefunden. In Stufe 2 werden insgesamt 3 bis 6 Gew.-% polares Lösemittel (die Mengenangabe schließt die Stufe 1 dabei ein) zugegeben. Bei einer anschließend durchgeführten Ultrafiltration findet man Phosphatidylcholin im Permeat. In Stufe 3 wird schließlich auf 6 bis 10 Gew.-% polares Lösemittel aufgefüllt. Hierbei wird bei einer Ultrafiltration im Permeat Phosphatidylethanolamin gefunden. Im Retentat verbleibt zuletzt das Phosphatidylinositol. Durch die vorgeschaltete Entfernung der Sterolglucoside kann man nun reinere Fraktionen an Ceramiden und Phosphatidylinositol, Phosphatidylethanolamin und Phosphatidylcholin erhalten, als das bisher mit den Verfahren nach Stand der Technik üblich war.

Die erhaltenen Fraktionen werden anschließend von den Lösemitteln befreit und getrocknet. Dabei werden folgende Reinheiten gefunden:

| | |
|---|---|
| Ceramide | : größer 60 % |
| Phosphatidylethanolamin | : größer 80 % |
| Phosphatidylcholin | : größer 80 % |
| Phosphatidylinositol | : größer 80% |

### Beispiele

### Beispiel 1

### Modifizierung von Lecithingemischen mittels Enzymen

Zur Anreicherung der freien Sterole in Lecithingemischen wird wie folgt vorgegangen: Jeweils 5 g Soja-Lecithin werden mit 5 g VE-Wasser versetzt und mit den Enzymen gemäß Abbildung 1 versetzt:

**Abb.1.**

| **Enzyme mit amylase- bzw. glucosidase-Aktivität** | | | |
|---|---|---|---|
| **Enzym** | **Organismus** | **Hersteller** | **Aktivität** |
| Glucosidase-β | Mandeln | Sigma | 3,7 U/mg solid |
| Depol 40 L (Glucosidase) | Aspergillus sp. | Biocatalysts | 1200 U/g |
| Depol 220 L (a-Amylase) | Aspergillus oryzae | Biocatalysts | 25000 U/g |
| Novo 188 (Glucosidase) | Aspergillus niger | Novozymes | 250 U/g |
| Cellubric (Glucosidase) | Trichoderma longi. Aspergillus niger | Novozymes | 1350 U/g |
| a-Amylase | Porcine Pancreas | Sigma | 13,4 U/mg |

Für jeden Ansatz wurden pro Enzym 100 mg zu den wässrigen Lecithinmischungen gegeben. Auf Mikrotiterplatten wurden die Gemische je nach Temperaturoptimum (zwischen 20 - 45 °C) des entsprechenden Enzyms 72 h lang gerührt. Eine Glucose spezifische Analyse mittels Dünnschichtchromatographie wurde jeweils von der unteren Wasserphase und der oberen organischen Phase durchgeführt. Als Glucose spezifisches Reagenz wurde dabei Oricnol in Schwefelsäure verwendet. Dabei die Zusammensetzung gemäß Tabelle 1 detektiert:

**Tabelle 1**

| **Zusammensetzung** | | |
|---|---|---|
| **Enzymzugabe** | **obere Phase** | **untere Phase** |
| ohne | 4 verschiedene Glucoside | keine Glucoside |
| beta-Glucosidase | 2 verschiedene Glucoside | 2 verschiedene Glucoside |
| Depol 40 L | 3 verschiedene Glucoside | ein Glucosid |
| Depol 220 L | 4 verschiedene Glucoside | keine Glucoside |
| Novo 188 | 4 verschiedene Glucoside | keine Glucoside |
| Cellubrix | 3 verschiedene Glucoside | ein Glucosid |
| alpha-Amlyase | 2 verschiedene Glucoside | 2 verschiedene Glucoside |
| Gemisch alpha Amylase/beta Glucosidase | Glucoside schwach erkennbar | 3 verschiedene Glucoside |

Erkennbar ist, dass durch eine Enzym-Mischung alpha-Amylase/beta-Glukosidase die beste Glucosid-Aufbrechung der Sterolglucoside gelungen ist.

### Beispiel 2

### Ultrafiltration mit nachgeschalteter Diafiltration vorbehandelter Lecithine

Die nachfolgende Versuch wurde in einer Ultrafiltrationsanlage mit 0,2 m² Membranfläche (Membrantyp UFM 2242 (MC = 2 x 10⁵) durchgeführt. Das maximale Feedvolumen betrug 40 L, die Betriebstemperatur maximal 20 bis 50 °C, der Betriebsdruck 2 bis 10 bar und der Volumenstrom bei 40 bar maximal 1200 1/h. Als Rohstoff wurde Lecithin aus Beispiel 1, das mit alpha-Amylase/beta-Glucosidase versetzt wurde, eingesetzt. Die Konzentration der Feedlösung betrug 5 Gew.-% Lecithin in n-Hexan. Die Versuchsdurchführung erfolgte im Batchbetrieb, d.h. das Konzentrat wurde in den Vorlage-/Arbeitsbehälter zurückgeführt. Es wurden die Betriebsparameter gemäß Tabelle 2 eingestellt. Unter jeweils festen Versuchsbedingungen (Druck, Volumenstrom, Temperatur) wurden verschiedene Konzentrationsstufen über "Haltepunkte" durchfahren.

**Tabelle 2**

| **Betriebsparameter** | | | |
|---|---|---|---|
| | **Druck** | **Temperatur** | **Umwälzung** |
| | **[bar]** | **[°C]** | **[l/h]** |
| **Soja-Lecithin** | 2 | 25 | 1200 |
| | 3 | 25 | 1200 |
| | 5 | 25 | 1200 |
| | 10 | 25 | 1200 |
| **Soja-Lecithin** | 3 | 40 | 1200 |
| | 5 | 40 | 1200 |
| | 10 | 40 | 1200 |

Das Permeat wurde so lange im Behälter zurück gefahren, bis der spezifische Permeatvolumenstrom NP (l/m²h) - definiert als Quotient aus dem Volumen des abgenommenen Permeats (1) und dem Produkt aus Permeationszeit (h) und der Membranfläche (m²) - konstant war. Dafür wurde im Abstand von 15 min der Permeatvolumenstrom gemessen. Anschließend wurde das Permeat abgenommen, bis man den nächsten Haltepunkt erreicht hatte (vgl. Tabelle 3).

**Tabelle 3**

| **Aufkonzentrierungsstufen** | | |
|---|---|---|
| **Konz. Faktor** | **Volumen Retentat** | **Volumen Permeat** |
| | **[l]** | **[l]** |
| 1 | 33,0 | - |
| 2 | 16,0 | 17,0 |
| 3 | 10,7 | 5,3 |
| 4 | 8,0 | 2,7 |
| 5 | 6,4 | 1,6 |
| 6 | 5,3 | 1,1 |
| 7 | 4,6 | 0,8 |

Haltepunkte sind charakterisiert durch die jeweilige Permeatausbeute bzw. den volumentrischen Konzentrierungsfaktor - definiert als Quotient aus dem vorgelegten, zu filtrierenden Volumen (1) und dem Volumen des Konzentrates (1). Man gelangt von einem Haltepunkt zum nächsten durch eine definierte Permeatabnahme. Anschließend wurde die Diafiltration durchgeführt. Es wurde n-Hexan im Volumenverhältnis 1:1 auf das Konzentrat zugegeben und über die Membran abgetrennt. Das Prozedere wurde drei- bzw. sechsmal wiederholt.

Die Ergebnisse der Ultra- und Diafiltration wurden mittels Dünnschichtchromatographie überprüft. Dabei zeigte sich, dass in das Permeat größer 97 % unpolare Lipide wie Fettsäuren, Triglyceride und Sterole gelangt sind. Im Retentat verbleiben die polaren Lipidstrukturen, wie Phosphatidylcholin, Phosphatidylethanolamin, Phosphatidylinositol und Ceramid-Strukturen.

### Beispiel 3

### Gewinnung von Sterolen aus dem Permeat

Zunächst wurde das n-Hexan mittels eines Rotationsverdampfers abgetrennt und das Gemisch so von Lösemittel befreit. Eine Analyse des Gemisches zeigte, dass sich 5 bis 7 Gew.-% freie Sterole in dem Gemisch befinden. Diese wurden auf zwei Wegen aufgearbeitet:

Variante 1: 100 g des getrockneten Gemischs wurden mit 100 g 5 molarer Natronlauge versetzt auf 90 °C aufgeheizt, 3 Stunden gerührt und somit komplett verseift. Nach Abkühlen auf Raumtemperatur wurde das Gemisch dreimal mit 80 g n-Heptan extrahiert. Dabei gingen die freien Sterole in die organische Phase über. Nach Beendigung der Extraktion wurde 70 Gew.-% des n-Heptans mittels Vakuum bei 50 °C entfernt. Anschließend wurden die Sterole bei 10 °C auskristallisiert, abfiltriert und mit frischem n-Heptan gewaschen. Die Ausbeute betrug 85 % der Theorie wobei die Reinheit der Sterole bei größer 95 % lag.

Variante 2: 400 g des getrockneten Gemischs wurden mit 80 g Methanol und mit 0,5 Gew.-% Zinkseife (bezogen auf Gesamtgemisch) versetzt und im Autoklaven auf 220 °C aufgeheizt. Nach 3 Stunden war die Reaktion beendet. Nach Abkühlen auf Raumtemperatur wurde das Gemisch im Rotationsverdampfer mittels Vakuum bei 80°C von Spuren von Wasser und Methanol befreit. Anschließend wurde das Gemisch über eine Kolonne fraktioniert. Dabei wurden bei 160 °C und 5 mbar 50 Gew.-% des Methylesters entfernt. Der Sumpf, der Methylester und Sterole enthielt, wurde anschließend auf 10 °C gekühlt und die Sterole bei 10 °C auskristallisiert, abfiltriert und mit frischem n-Heptan gewaschen. Die Ausbeute betrug 80 % der Theorie wobei die Reinheit der Sterole bei größer 90 % lag.

### Beispiel 4

### Gewinnung von Phospholipiden aus dem Retentat

Das Retentat aus Beispiel 2 wurde zunächst mittels eines Rotationsverdampfer bei 10 mbar und 50 °C vom n-Hexan befreit. Eine GC-Headspace Analyse zeigte, dass anschließend n-Hexan noch in einer Menge kleiner 1 ppm im Gemisch vorhanden war, während der Anteil an Phospholipiden etwa 97 Gew.-% betrug.

### Beispiel 5

### Fraktionierung der Phospholipide aus dem Retentat

Das Retentat aus Beispiel 2 wurde mit 0,5 Gew.-% Ethanol versetzt. Anschließend wurde eine Ultrafiltration analog Beispiel 2 durchgeführt. Im Permeat I fanden sich nach Entfernung des Lösungsmittels 62 Gew.-% Stoffe mit Ceramidstruktur, die mittels Dünnschichtchromatographie (DC) nachgewiesen werden. Dem erhaltenen Retentat I wurden weitere 3,5 Gew.-% Ethanol zugegeben. Anschließend wurde eine Ultrafiltration analog Beispiel 2 durchgeführt. Im Permeat II fanden sich nach Entfernung des Lösemittelgemisches 83 Gew.-% Phosphatidylcholine, welches ebenfalls mittels DC nachgewiesen wurden. Dem so erhaltenen Retentat II wurden weitere 5 Gew.-% Ethanol zugegeben. Anschließend wurde eine Ultrafiltration analog Beispiel 2 durchgeführt. Im Permeat III fanden sich nach Entfernung des Lösemittelgemisches 83 Gew.-% Phosphatidylethanolamine, welche wiederum mittels DC nachgewiesen wurden. Das so erhaltene Retentat III wurde ebenfalls vom Lösemittel entfernt. Es wurden 86 Gew.-% einer Phosphatidylinositolfraktion gefunden, wobei der Nachweis wiederum mittels DC erfolgte.

### Vergleichsbeispiel V1

### Aufarbeitung eines unbehandelten Lecithins mittels Ultra- und Diafiltration

Der nachfolgende Versuch wurde analog Beispiel 2 durchgeführt. Die Konzentration der Feedlösung betrug 5 Gew.-% Lecithin in n-Hexan. Die Versuchsdurchführung erfolgte im Batchbetrieb, d.h. das Konzentrat wurde in den Vorlage-/Arbeitsbehälter zurückgeführt. Es wurden die Betriebsparameter gemäß Tabelle 2, Beispiel 2 eingestellt. Das Permeat wurde abgenommen, bis man den nächsten Haltepunkt erreicht hatte (vgl. Tabelle 3, Beispiel 2). Anschließend wurde die Diafiltration durchgeführt. Es wurde n-Hexan im Volumenverhältnis 1:1 auf das Konzentrat zugegeben und über die Membran abgetrennt. Das Prozedere wurde drei- bzw. sechsmal wiederholt. Die Ergebnisse der Ultra- und Diafiltration wurden mittels Dünnschichtchromatographie überprüft. Dabei zeigte sich, dass in das Permeat größer 97 % unpolare Lipide wie Fettsäuren, Triglyceride und Sterole gelangt sind. Im Retentat verblieben die polaren Lipidstrukturen, wie Phosphatidylcholin, Phosphatidylethanolamin, Phosphatidylinositol und Ceramid-Strukturen sowie Sterolglucoside, die mittels einer Glucose spezifischen DC-Analyse nachgewiesen wurden.

### Vergleichsbeispiel V2

### Gewinnung von Sterolen aus dem Permeat eines unbehandelten Lecithins aus V1

Im ersten Schritt wurde das Permeat mittels eines Rotationsverdampfers vom Lösemittel befreit; die Analyse des Gemisches zeigte, dass sich nur 1 bis 2 Gew.-% freie Sterole in dem Gemisch befanden. Diese werden nach zwei Wegen aufgearbeitet:
Variante 1: 100 g des getrockneten Gemischs wurden mit 100 g 5 molarer Natronlauge versetzt auf 90 °C aufgeheizt, 3 Stunden gerührt und somit komplett verseift. Nach Abkühlen auf Raumtemperatur wurde das Gemisch dreimal mit 80 g n-Heptan extrahiert. Dabei gingen die freien Sterole in die organische Phase über. Nach Beendigung der Extraktion wurden 70 Gew.-% des n-Heptans mittels Vakuum bei 50 °C entfernt. Anschließend wurden die Sterole bei 10 °C auskristallisiert, abfiltriert und mit frischem n-Heptan gewaschen. Die Ausbeute betrug 81 % der Theorie, die Reinheit der Sterole lag bei 95 %. In diesem Beispiel wurde die Ausbeute um einen Faktor größer 5 an Sterolen reduziert.
Variante 2: 400 g des getrockneten Gemischs wurden mit 80 g Methanol, mit 0,5 Gew.-% Zinkseife (bezogen auf Gesamtgemisch) versetzt und im Autoklaven auf 220 °C aufgeheizt. Nach 3 Stunden war die Reaktion beendet. Nach Abkühlen auf Raumtemperatur wurde das Gemisch im Rotationsverdampfer mittels Vakuum bei 80 °C von Spuren von Wasser und Methanol befreit. Anschließend wurde das Produkt über eine Kolonne fraktioniert. Dabei wurden bei 160 °C und 5 mbar 50 Gew.-% des Methylesters entfernt. Der Sumpf, der Methylester und Sterole enthielt, wurde anschließend auf 10 °C gekühlt und die Sterole bei 10 °C auskristallisiert, abfiltriert und mit frischem n-Heptan gewaschen. Die Ausbeute betrug 75 % der Theorie, die Reinheit der Sterole lag bei 90 %. In diesem Beispiel wurde die Ausbeute um einen Faktor größer 5 an Sterolen reduziert.

### Vergleichsbeispiel V3

### Fraktionierung der Phospholipide aus dem Retentat eines unbehandelten Lecithins aus V1

Das Retentat aus Beispiel 2 wurde mit 0,5 Gew.-% Ethanol versetzt. Anschließend wurde einer Ultrafiltration analog Beispiel 2 durchgeführt. Im Permeat I fanden sich nach Entfernung des Lösemittels 30 Gew.-% Stoffe mit Ceramidstruktur, die mittels DC nachgewiesen werden. Dem erhaltenen Retentat I wurden weitere 3,5 Gew.-% Ethanol zugegeben. Anschließend wurde eine Ultrafiltration analog Beispiel 2 durchgeführt. Im Permeat II fanden sich nach Entfemung des Lösegemisches 30 Gew.-% Phosphatidylcholine, welches mittels DC nachgewiesen wurden. Dem erhaltenen Retentat II wurden weitere 5 Gew.-% Ethanol zugegeben. Anschließend wurde eine Ultrafiltration analog Beispiel 2 durchgeführt. Im Permeat III fanden sich nach Entfernung des Lösemittelgemisches 30 Gew.-% Phosphatidylethanolamine, welche mittels DC nachgewiesen wird. Das resultierende Retentat III wurde ebenfalls vom Lösemittel befreit Es werden 40 Gew.-% einer Phosphatidylinositole gefunden, die wiederum mittels DC nachgewiesen wurden.

## Patentansprüche

1. Verfahren zur gleichzeitigen Herstellung von Sterolen und polaren Lipiden, bei dem man Zubereitungen pflanzlicher Lecithine einer Ultrafiltration unterwirft und einerseits die Sterole im Permeat und andererseits die polaren Lipide im Retentat anreichert und anschließend in an sich bekannter Weise aufarbeitet **dadurch gekennzeichnet, dass** man wässrige Lecithinzubereitungen zuvor mit Enzymen behandelt, die eine Amylase-und Glucosidase-Aktivität aufweisen.

2. Verfahren nach Anspruch 1, **dadurch gekennzeichnet, dass** man als polare Lipide Ceramide und Phospholipide herstellt.

3. Verfahren nach den Ansprüchen 1 und/oder 2, **dadurch gekennzeichnet, dass** man Lecithinlösungen einsetzt, die durch Entschleimung von pflanzlichen Ölen gewonnen werden

4. Verfahren nach Anspruch 3, **dadurch gekennzeichnet, dass** man pflanzliche Öle entschleimt, die ausgewählt sind aus der Gruppe, die gebildet wird von Sojaöl, Rapsöl, Sonnenblumenöl, Leinöl, Linolaöl, Distelöl, Olivenöl und deren Gemischen.

5. Verfahren nach mindestens einem der Ansprüche 1 bis 4, **dadurch gekennzeichnet, dass** man die Lecithine mit unpolaren organischen Lösemitteln versetzt.

6. Verfahren nach Anspruch 7, **dadurch gekennzeichnet, dass** man als organisches Lösemittel n-Hexan einsetzt.

7. Verfahren nach mindestens einem der Ansprüche 1 bis 6, **dadurch gekennzeichnet, dass** man 1 bis 10 Gew.-%ige Lösungen von Lecithin in einem unpolaren organischen Lösemittel einsetzt.

8. Verfahren nach mindestens einem der Ansprüche 1 bis 7, **dadurch gekennzeichnet, dass** man der Ultrafiltration eine Diafiltration nachschaltet.

9. Verfahren nach mindestens einem der Ansprüche 1 bis 8, **dadurch gekennzeichnet, dass** man die Ultrafiltration bzw. die Kombination aus Ultra- und Diafiltration bei Temperaturen im Bereich von 20 bis 50 °C durchführt.

## Claims

1. A process for the simultaneous production of sterols and polar lipids, in which preparation of vegetable lecithins are subjected to ultrafiltration and, on the one hand, the sterols in the permeate and, on the other hand, the polar lipids in the retentate are enriched, followed by working up by methods known per se, **characterized in that** aqueous lecithin preparations are pretreated with enzymes with amylase or glucosidase activity.

2. A process as claimed in claim 1, **characterized in that** ceramides and phospholipids are produced as polar lipids.

3. A process as claimed in claims 1 and/or 2, **characterized in that** lecithin solutions obtained by degumming of vegetable oils are used.

4. A process as claimed in claim 3, **characterized in that** vegetable oils selected from the group consisting of soybean oil, rapeseed oil, sunflower oil, linseed oil, linola oil, thistle oil, olive oil and mixtures thereof are degummed.

5. A process as claimed in at least one of claims 1 to 4, **characterized in that** nonpolar organic solvents are added to the lecithins.

6. A process as claimed in claim 5 charcterized in that n-hexane in used as the organic solvent.

7. A process as claimed in at least one of claims 1 to 6 **characterized in that** 1 to 10% by weight solutions of lecithin in a nonpolar organic solvent are used.

8. A process as claimed in at least one of claims 1 to 7, **characterized in that** a diafiltration is conducted after the ultrafiltration.

9. A process as claimed in at least one of claims 1 to 8, charcterized in that, the ultrafiltration or the combination of ultra- and diafiltration is conducted in a temperature range from 20 to 50°C.

## Revendications

1. Procédé pour la production simultanée de stérols et de lipides polaires, dans lequel on soumet des préparations de lécithines végétales à une ultrafiltration et on concentre d'une part les stérols dans le perméat et d'autre part les lipides polaires dans le rétentat et ensuite on effectue un traitement final d'une façon connue en soi, **caractérisé en ce qu'**on traite au préalable les préparations aqueuses de lécithine par des enzymes qui présentent une activité amylase et une activité glucosidase.

2. Procédé selon la revendication 1, **caractérisé en ce qu'**en tant que lipides polaires on obtient des céramides et des phospholipides.

3. Procédé selon la revendication 1 et/ou la revendication 2, **caractérisé en ce qu'**on utilise des solutions de lécithine qui sont obtenues par démucilagination d'huiles végétales.

4. Procédé selon la revendication 3, **caractérisé en ce qu'**on démucilaginise des huiles végétales qui sont choisies dans le groupe constitué par l'huile de soja, l'huile de colza, l'huile de tournesol, l'huile de lin, l'huile de linola, l'huile de carthame, l'huile d'olive et des mélanges de celles-ci.

5. Procédé selon au moins l'une des revendications 1 à 4, **caractérisé en ce qu'**on ajoute aux lécithines des solvants organiques non polaires.

6. Procédé selon la revendication 5, **caractérisé en ce que** comme solvant organique on utilise le n-hexane.

7. Procédé selon au moins l'une des revendications 1 à 6, **caractérisé en ce qu'**on utilise des solutions de lécithine à 1-10 % en poids dans un solvant organique non polaire.

8. Procédé selon au moins l'une des revendications 1 à 7, **caractérisé en ce qu'**on fait suivre l'ultrafiltration d'une diafiltration.

9. Procédé selon au moins l'une des revendications 1 à 8, **caractérisé en ce qu'**on effectue l'ultrafiltration ou la combinaison d'ultrafiltration et de diafiltration à des températures dans la plage de 20 à 50 °C.
